# EUROPEAN PATENT APPLICATION

(11) **EP 4 365 169 A1**
(43) Date of publication of application: **08.05.2024**
(21) Application number: 22856269.0
(22) Date of filing: 12.08.2022
(51) Int. Cl.: C07D 327/04, C07D 327/06, H01M 10/0567

(54) **COMPOUND AND PREPARATION METHOD THEREOF**

(30) Priority: 13.08.2021 KR 20210107249
(71) Applicant: LG Chem, Ltd., Yeongdeungpo-gu Seoul 07336 (KR); LG Energy Solution, Ltd., Seoul 07335 (KR)
(72) Inventor: LEE, Mi Sook, Daejeon 34122 (KR); YOON, Jeong Ae, Daejeon 34122 (KR); KIM, Su Jeong, Daejeon 34122 (KR); LEE, Chul Haeng, Daejeon 34122 (KR); LEE, Jung Min, Daejeon 34122 (KR); YEOM, Chul Eun, Daejeon 34122 (KR)
(74) Representative: Hoffmann Eitle
(86) International application number: PCT/KR2022/012080
(87) International publication number: WO 2023/018276

(57) **Abstract**

The present invention relates to a precursor compound which can be used when preparing an annular sulfonic acid ester derivative compound, and a preparation method thereof.

## Description

### TECHNICAL FIELD

### Cross-reference to Related Applications

The present application claims the benefit of priority based on Korean Patent Application No. 10-2021-0107249, filed on August 13, 2021, all the disclosures of which are incorporated by reference herein.

### Technical Field

The present invention relates to a precursor compound of a cyclic sulfonic acid ester derivative compound, and a method for preparing same.

### BACKGROUND ART

1,3-Propane sultone and 1,4-butane sultone are known as useful nonaqueous electrolyte additives of a lithium secondary battery. However, 1,3-propane sultone and 1,4-butane sultone have problems concerning toxic issues, gas production, stability, or the like. Accordingly, research on a cyclic sulfonic acid ester derivative compound which may be used as an additive in a nonaqueous electrolyte instead of 1,3-propane sultone and 1,4-butane sultone, is being actively conducted. For example, research on a cyclic sulfonic acid ester derivative compound introducing a derivative at the gamma position of 1,3-propane sultone (carbon position beside oxygen in a ring structure) or a cyclic sulfonic acid ester derivative compound introducing a derivative at the delta position of 1,4-butane sultone (carbon position beside oxygen in a ring structure), that may, if used as the additive of the nonaqueous electrolyte of a secondary battery, form an electrode-electrolyte interface that is stable at a high temperature and has low resistance, and may improve the life characteristics of a lithium secondary battery, is being actively conducted.

Concerning this, in order to easily prepare the cyclic sulfonic acid ester derivative compound introducing a derivative at the gamma position of 1,3-propane sultone or the cyclic sulfonic acid ester derivative compound introducing a derivative at the delta position of 1,4-butane sultone, that is, in order to easily prepare a cyclic sulfonic acid ester derivative compound, research on a compound used as a precursor for preparing the derivative compound is required.

### DISCLOSURE OF THE INVENTION

### TECHNICAL PROBLEM

The present invention provides a compound used as a precursor for preparing a cyclic sulfonic acid ester derivative compound, and a method for preparing same.

### TECHNICAL SOLUTION

In order to solve the above-described tasks, the present invention provides a compound and a method for preparing same.
(1) The present invention provides a compound represented by Formula I. 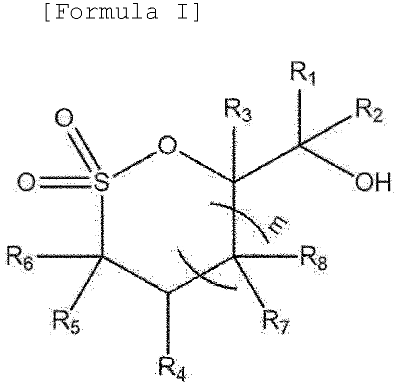
   In Formula I,
   m is 0 or 1, and
   R₁ to R₈ are each independently hydrogen; or a substituted or unsubstituted C₁-C₁₀ alkyl group.
(2) The present invention provides the compound according to (1) above, wherein R₁ to R₈ are each independently hydrogen; or an unsubstituted C₁-C₆ alkyl group.
(3) The present invention provides the compound according to (1) or (2) above, wherein the compound represented by Formula I is a compound represented by Formula a or b. 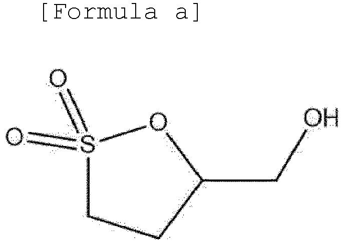 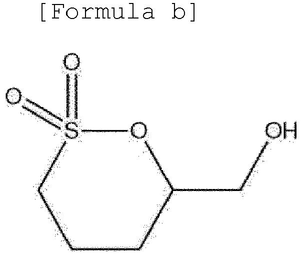
(4) In addition, the present invention provides a method for preparing the compound represented by Formula I, comprising: (A) a step of reacting a compound represented by Formula 1 with sodium metabisulfite or sodium hydrogen sulfite to prepare a compound represented by Formula 2; (B) a step of reacting the compound represented by Formula 2 with thionyl halide to prepare a compound represented by Formula 3; and (C) a step of hydrolyzing the compound represented by Formula 3 to prepare the compound represented by Formula I. 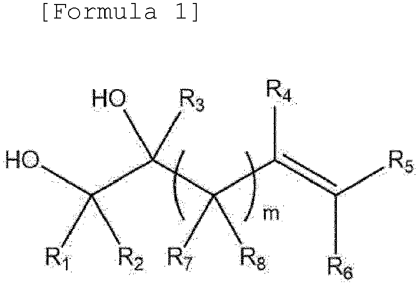 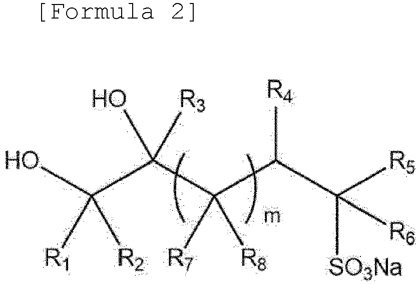 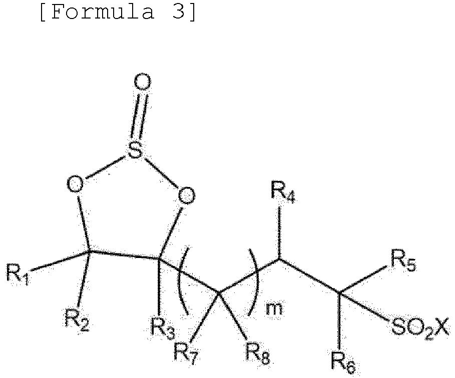
   In Formula 1 to Formula 3,
   m is 0 or 1,
   R₁ to R₈ are each independently hydrogen; or a substituted or unsubstituted C₁-C₁₀ alkyl group, and
   X is halogen.
(5) The present invention provides the method for preparing the compound according to (4) above, wherein R₁ to R₈ are each independently hydrogen; or an unsubstituted C₁-C₆ alkyl group.
(6) The present invention provides the method for preparing the compound according to (4) or (5) above, wherein step (A) is performed under pH 7.0-7.5.
(7) The present invention provides the method for preparing the compound according to any one of (4) to (6) above, wherein step (A) is performed at 60°C to 70°C.
(8) The present invention provides the method for preparing the compound according to any one of (4) to (7) above, wherein step (B) is performed at 60°C to 70°C.
(9) The present invention provides the method for preparing the compound according to any one of (4) to (8) above, wherein step (B) is performed at room temperature.

### ADVANTAGEOUS EFFECTS

By using the compound represented by Formula I according to the present invention, a derivative may be stably introduced at a carbon position beside oxygen in the ring structure of a cyclic sulfonic acid ester derivative compound.

### BRIEF DESCRIPTION OF DRAWINGS

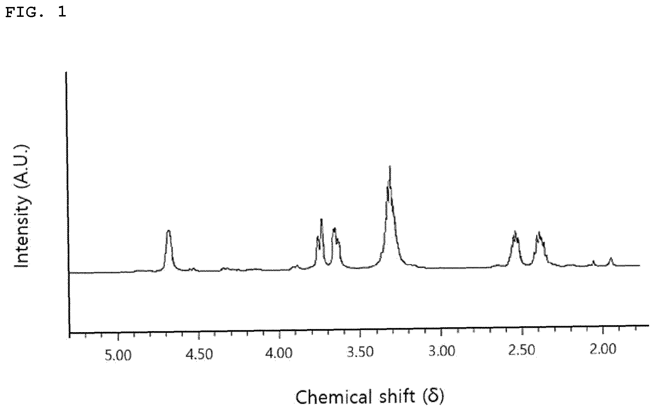

FIG. 1 is a ¹H-NMR spectrum of a compound represented by Formula a.

### BEST MODE FOR CARRYING OUT THE INVENTION

Hereinafter, the present invention will be described in more detail.

It will be understood that words or terms used in the description and claims of the present invention shall not be interpreted as the meaning defined in commonly used dictionaries. It will be understood that the words or terms should be interpreted as having a meaning that is consistent with their meaning in the technical idea of the invention, based on the principle that an inventor may properly define the meaning of the words to best explain the invention.

The terms used in the specification are used for only explaining example embodiment and do not intend to limit the present invention. The singular forms are intended to include the plural forms as well, unless the context clearly indicates otherwise. It will be further understood that the terms "comprises", "comprising", "having", or the like, used in this specification, specify the presence of stated features, numerals, steps, elements, or the combination thereof, but do not preclude the presence or addition of one or more other features, numerals, steps, elements, or the combination thereof.

### Compound represented by Formula I

The present invention provides a compound represented by Formula I. 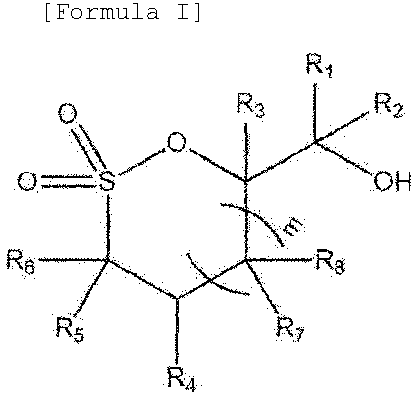
In Formula I,
m is 0 or 1, and
R₁ to R₈ are each independently hydrogen, or a substituted or unsubstituted C₁-C₁₀ alkyl group.

In the present invention, in a substituted alkyl group, a substituent may be deuterium, a halogen group, a hydroxyl group, an amino group, a thiol group, a cyano group, or a linear or branched C₁-C₆ alkoxy group.

The compound represented by Formula I is the precursor of a cyclic sulfonic acid ester derivative compound, and a derivative may be stably introduced at a carbon position beside oxygen in the ring structure of the cyclic sulfonic acid ester derivative compound.

According to the present invention, in terms of easy synthesis of Formula I, R₁ to R₈ may be each independently hydrogen; or an unsubstituted C₁-C₆ alkyl group. Particularly, R₁ to R₈ may be all hydrogen in terms of easy synthesis and structural stability.

Meanwhile, if m is 0, it could be more favorable in terms of reduction decomposition.

According to the present invention, the compound represented by Formula I may be a compound represented by Formula a or b. The compound represented by Formula I may particularly be a compound represented by Formula a.

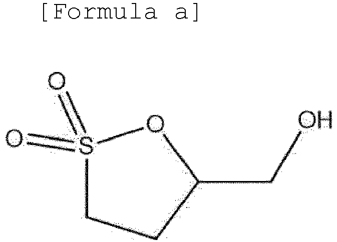

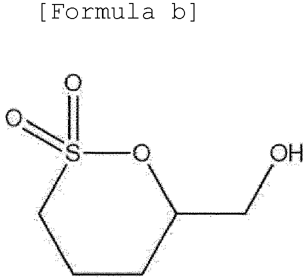

Meanwhile, the compound represented by Formula I may be used as a precursor for preparing a cyclic sulfonic acid ester derivative compound. For example, the compound represented by Formula I may be used as a precursor for preparing compound of Formula II. 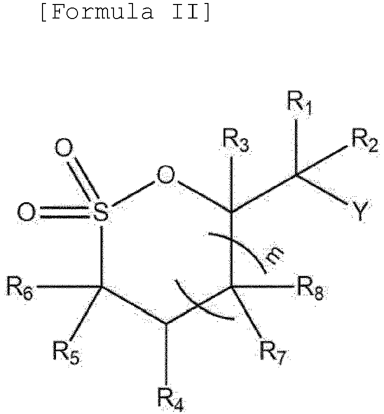
In Formula II,
m is 0 or 1, and
R₁ to R₈ are each independently hydrogen; or a substituted or unsubstituted C₁-C₁₀ alkyl group,
Y is -SO₂R', -SO₃R', -OC(=O)NHR', -OC(=S)NHR', and
R' is a lithium salt or a substituted or unsubstituted C₁-C₁₀ alkyl group.

The compound represented by Formula II may be prepared by i) reacting the hydroxyl group of the compound represented by Formula I with SOCl₂ to form sulfonyl chloride, and then, reacting with an alkyl-alcohol, fluoro-alcohol or cyano-alcohol, or the like, or prepared by ii) reacting the hydroxyl group of the compound represented by Formula I with a compound including a halogen atom, an isocyanate or thiocyanate functional group, or the like.

If the compound represented by Formula II is included in a nonaqueous electrolyte, a thin and stable SEI layer may be formed, and accordingly, a lithium secondary battery having stability at a high temperature and excellent life characteristics may be provided.

### Method for preparing compound represented by Formula I

In addition, the present invention provides a method for preparing the compound represented by Formula I, comprising: (A) a step of reacting a compound represented by Formula 1 with sodium metabisulfite or sodium hydrogen sulfite to prepare a compound represented by Formula 2; (B) a step of reacting the compound represented by Formula 2 with thionyl halide to prepare a compound represented by Formula 3; and (C) a step of hydrolyzing the compound represented by Formula 3 to prepare the compound represented by Formula I. 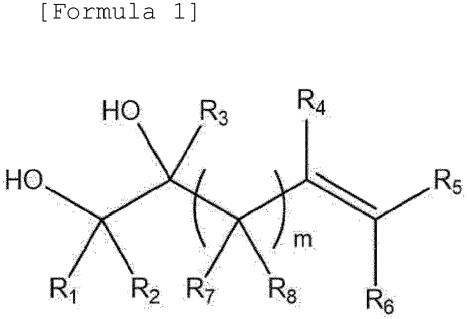 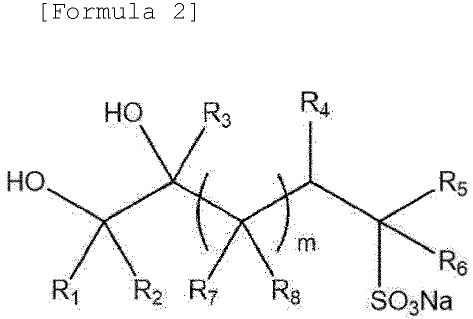 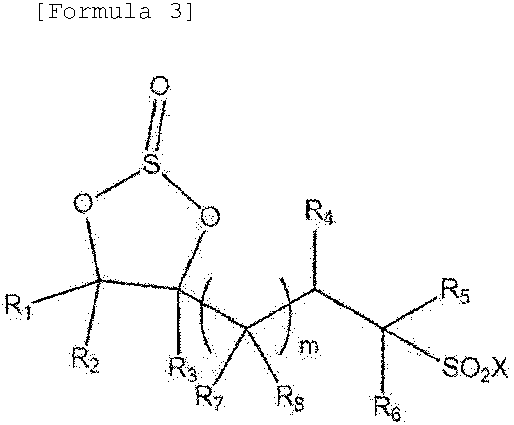
In Formula 1 to Formula 3,
m is 0 or 1,
R₁ to R₈ are each independently hydrogen; or a substituted or unsubstituted C₁-C₁₀ alkyl group, and
X is a halogen group.

According to the present invention, R₁ to R₈ are each independently hydrogen; or an unsubstituted C₁-C₆ alkyl group in terms of easy synthesis. Particularly, R₁ to R₈ may be all hydrogen in terms of easy synthesis and structural stability.

Step (A) is a step of adding sodium hydrogen sulfite to the alkene of the compound represented by Formula 1. In this case, pH may be set so that the sodium hydrogen sulfite may be added to carbon to which R₅ and R₆ are bonded to produce a main product (the compound represented by Formula 2).

According to the present invention, step (A) may be performed under pH 7.0-7.5, particularly, pH 7.0-7.3 to produce the compound represented by Formula 2 as a main product, and the pH may be controlled by sodium hydroxide.

According to the present invention, step (A) may be performed at 60°C to 70°C. Step (A) may particularly be a step of reacting the compound represented by Formula 1 with sodium metabisulfite or sodium hydrogen sulfite at 60°C to 70°C for 5 hours to 10 hours, and water may be used as a solvent.

Step (B) is a step of forming a ring including -O-S(=O)-O- of the compound represented by Formula 3 by two hydroxyl groups of the compound represented by Formula 2.

According to the present invention, step (B) may be performed at 60°C to 70°C. Step (B) may particularly be a step of reacting the compound represented by Formula 2 with thionyl halide at 60°C to 70°C for 5 hours to 10 hours, and a nonpolar solvent such as CHCl₃ may be used as a solvent. Meanwhile, the thionyl halide of step (B) may be thionyl fluoride or thionyl chloride. In addition, step (B) may be performed under a catalyst such as N,N-dimethylformamide.

Step (C) is a step of injecting an alcohol or water to Formula 3 that is an intermediate to undergo a hydrolysis process to form a sultone structure.

According to the present invention, step (C) may be performed at room temperature, for example, at 20°C to 30°C. Step (C) may be a step of reacting the compound represented by Formula 3 with water or an alcohol at room temperature for 1 hour to 5 hours, and the alcohol may be, for example, methanol, ethanol, or the like.

### MODE FOR CARRYING OUT THE INVENTION

Hereinafter, a preferred embodiment will be suggested to assist the understanding of the present invention. However, the embodiment is only for illustrating the present invention, and various changes and modifications may be possible within the scope and technical range of the present description. Such changes and modifications are of course possible within the claims attached herewith.

### Example

### Example 1. Preparation of compound represented by Formula a

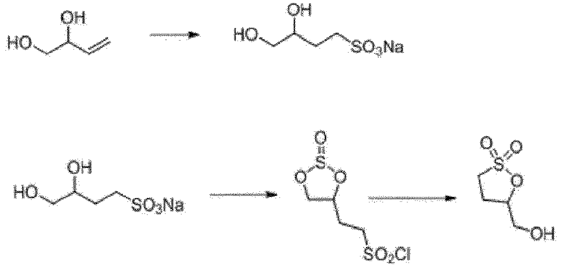

32 g (368 mmol) of 3-butene-1,2-diol was dissolved in 200 ml of DI water, and 76 g (404 mmol) of Na₂S₂O₅ was injected thereto. Sodium hydroxide was added thereto, and the reaction was performed at 65°C for 7 hours while maintaining pH to a level of 7.0. After finishing the reaction, the reaction solution was cooled to room temperature, and distillation under a reduced pressure was performed. If the solution started to be milky and opaque, the distillation under a reduced pressure was stopped, and methanol was injected. Then, the reaction solution was filtered, and the filtrate solution was distilled under a reduced pressure to precipitate a white solid. The precipitate was dried in a vacuum oven of 60°C, to obtain 77 g (purity: 90%) of sodium 3,4-dihydroxybutane sulfonate.

30 g (160 mmol, purity 90%) of 3,4-sodium dihydroxybutane sulfonate was suspended in 200 ml of CHCl₃, 0.58 g (10 mmol, Aldrich Co.) of N,N-dimethylformamide (DMF) was added thereto, and 37.57 g (320 mmol, Aldrich Co.) of thionyl chloride was added little by little dropwisely, followed by reacting at 65°C for 7 hours. After finishing the reaction, the reaction solution was cooled to room temperature and filtered, and the filtrate solution was concentrated under a reduced pressure.

To the solution concentrated under a reduced pressure, 50 g (1.57 mol, Aldrich Co.) of methanol was added, and reaction was performed at room temperature for 2 hours.

After finishing the reaction, the reaction solution was concentrated under a reduced pressure. To the solution thus concentrated, 50 ml of ethyl acetate and 30 ml of water were added and stirred. An organic layer (ethyl acetate layer) was extracted, washed with water and then concentrated again to obtain 20 g (yield: 83%) of a compound represented by Formula a (5-(hydroxymethyl)-1,2-oxathiolan-2,2-dioxide) as a transparent liquid.

¹H-NMR data of the compound represented by Formula a are as follows, and a ¹H-NMR spectrum is shown in FIG. 1.

¹H-NMR (Acetonitrile-d3): δ 4.67(1H), δ 3.6-3.8(2H), δ 3.2-3.4(2H), δ 2.3-2.6(2H)

## Claims

1. A compound represented by Formula I: 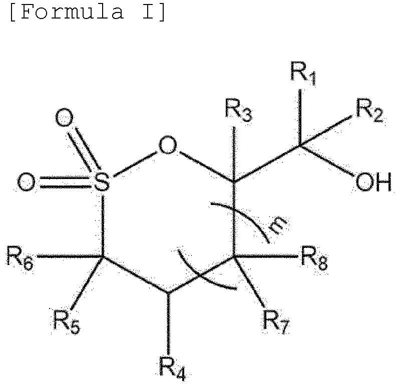
in Formula I,
m is 0 or 1, and
R₁ to R₈ are each independently hydrogen; or a substituted or unsubstituted C₁-C₁₀ alkyl group.

2. The compound according to claim 1, wherein R₁ to R₈ are each independently hydrogen; or an unsubstituted C₁-C₆ alkyl group.

3. The compound according to claim 1, which is represented by Formula a or b:

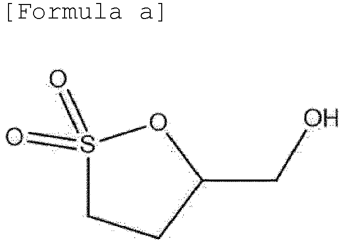

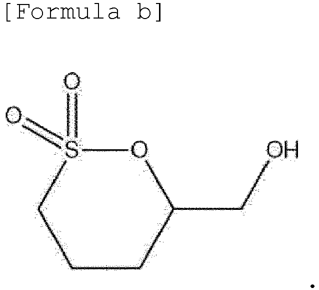

4. A method for preparing the compound according to claim 1, the method comprising:
(A) reacting a compound represented by Formula 1 with sodium metabisulfite or sodium hydrogen sulfite to prepare a compound represented by Formula 2;
(B) reacting the compound represented by Formula 2 with thionyl halide to prepare a compound represented by Formula 3; and
(C) hydrolyzing the compound represented by Formula 3 to prepare the compound represented by Formula I: 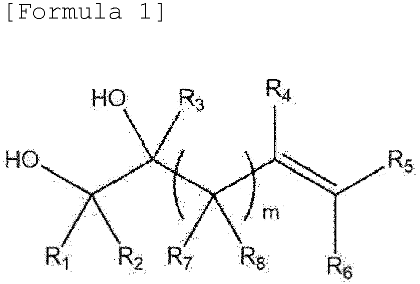 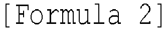 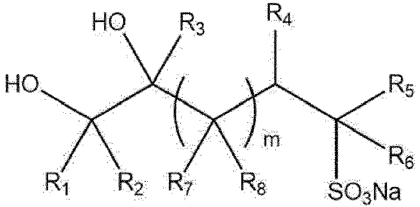 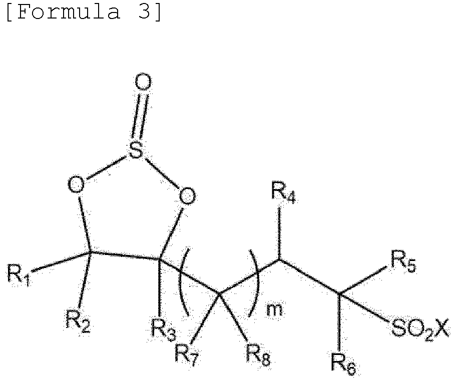
in Formula 1 to Formula 3,
m is 0 or 1,
R₁ to R₈ are each independently hydrogen; or a substituted or unsubstituted C₁-C₁₀ alkyl group, and
X is halogen.

5. The method for preparing the compound according to claim 4, wherein R₁ to R₈ are each independently hydrogen; or an unsubstituted C₁-C₆ alkyl group.

6. The method for preparing the compound according to claim 4, wherein step (A) is performed under pH 7.0-7.5.

7. The method for preparing the compound according to claim 4, wherein step (A) is performed at 60°C to 70°C.

8. The method for preparing the compound according to claim 4, wherein step (B) is performed at 60°C to 70°C.

9. The method for preparing the compound according to claim 4, wherein step (B) is performed at room temperature.
